# EUROPÄISCHE PATENTSCHRIFT

(11) **EP 2 246 006 B1**
(45) Veröffentlichungstag und Bekanntmachung des Hinweises auf die Patenterteilung: **13.07.2016**
(21) Anmeldenummer: 10004464.3
(22) Anmeldetag: 28.04.2010
(51) Int. Cl.: B25J 18/00, A61B 34/30

(54) **Operations-Assistenz-System**
Operations assistance system
Système d'assistance à l'opération

(30) Priorität: 28.04.2009 DE 102009018917
(43) Veröffentlichungstag der Anmeldung: 03.11.2010
(73) Patentinhaber: Aktormed GmbH, 93092 Barbing (DE)
(72) Erfinder: Geiger, Robert, Dipl.-Ing. (FH), 94516 Metten (DE); Kraus, Peter, 93059 Regensburg (DE)
(74) Vertreter: Glück Kritzenberger Patentanwälte PartGmbB

(56) Entgegenhaltungen:
- WO-A2-2007/041267
- DE-A1-102007 019 363
- FR-A1- 2 590 827
- GB-A- 2 133 770
- SELIGER G ET AL: "LEICHTBAUTECHNIKEN FUER GELENKARMROBOTER. ÖLIGHTWIEGHY CONSTRUCTIONAL TECHNIQUES FOR ROBOTS WITH ARTICULATED ARMS", ZWF ZEITSCHRIFT FUR WIRTSCHAFTLICHE FERTIGUNG UNDAUTOMATISIERUNG, CARL HANSER VERLAG. MUNCHEN, DE, Bd. 89, Nr. 4, 1. April 1994 (1994-04-01), Seiten 171-173, XP000441974, ISSN: 0947-0085

## Beschreibung

Die Erfindung bezieht sich auf ein Operations-Assistenz-System gemäß dem Oberbegriff des Patentanspruches 1.

Operations-Assistenz-Systeme, insbesondere zur Unterstützung von minimal invasiven Eingriffen oder Operationen sind hinlänglich bekannt. Diese werden beispielsweise zur Führung von Hilfsinstrumenten, wie z.B. Kamerasystemen usw. verwendet.

Aus der DE 10 2007 019 363 A1 ist beispielsweise ein Operations-Assistenz-System zur Führung von chirurgischen bzw. medizinischen Werkzeugen oder Instrumenten bekannt, welche eine Kinematik mit Instrumentenhalterung umfasst, mit der die Instrumentenhalterung und das daran befestigte chirurgische oder medizinische Werkzeug oder Instrument in mehreren Achsen motorisch und gesteuert bewegbar ist. Die Kinematik umfasst hierbei wenigstens eine Tragsäule, die um wenigstens eine Achse gesteuert an einer Basis des Operations-Assistenz-System vorgesehen ist, sowie einen ersten und zweiten Arm, von denen der erste äußere Arm an einem Ende die Instrumentenhalterung trägt und mit seinem anderen Ende mit dem einem Ende des zweiten Armes drehbeweglich verbunden ist. Das andere Ende des zweiten Armes ist mit einem dritten Arm ebenfalls drehbeweglich verbunden, wobei die Drehachse annähernd parallel zur Längsachse des ersten, die Instrumentenhalterung tragenden Armes verläuft. Nachteilig entsteht während des Operationsverlaufes bei der beschriebenen Kinematik eine Behinderung des Operateurs durch den nahezu parallel zum Patientenkörper angeordneten ersten Arm.

Aus der GB 2 133 770 A ist ein Schweißroboter mit zumindest einem äußeren und inneren Arm und einer Tragsäule bekannt, an dessen äußeren Arm ein Werkzeugträger vorgesehen ist. Auch aus der FR 2 590 827 A1 und der WO 2007/041267 A2 sind Industrierobotersysteme mit mehreren Armen bekannt.

Ausgehend davon ist es Aufgabe der Erfindung, ein Operations-Assistenz-System insbesondere auch für minimalinvasive Interventionen oder Operationen aufzuzeigen, welches eine verbesserte Kinematik aufweist, die dem Operateur einen verbesserten Zugang zur Oberseite des Patientenkörpers ermöglicht.

Die Aufgabe wird ausgehend von den Merkmalen des Oberbegriffes des Patentanspruches 1 durch dessen kennzeichnende Merkmale gelöst.

Ein wesentlicher Aspekt des erfindungsgemäßen Operations-Assistenz-System ist darin zu sehen, dass die Armlängsachse des äußeren Armes parallel oder zumindest näherungsweise parallel zur Längsachse des Instrumententrägers verläuft und zur drehbaren Verbindung des äußeren Armes mit dem Instrumententräger ein Drehgelenk vorgesehen ist, dass zur lösbaren Befestigung des Instrumententrägers am äußeren Armes ausgebildet ist. Besonders vorteilhaft wird durch die erfindungsgemäße Armführung eine für den Operateur äußerst zufriedenstellende Führung des Hilfsinstruments erreicht, die keinesfalls den Aktionsbereich des Operateurs einschränkt sowie darüber hinaus eine verbesserte Führung des Hilfsinstruments ermöglicht.

Weiterhin vorteilhaft sind der äußere Arm und/oder Instrumententräger im Querschnitt L-förmig ausgebildet.

Bei einer bevorzugten Ausführungsform der Erfindung sind sämtliche Arme des Systems bzw. der Kinematik dieses System jeweils als Hohlkörper ausgebildet. Zumindest der äußere Arm besteht dabei aus einem Werkstoff, der eine hohe Stabilität und Belastbarkeit gewährleistet, zur Erzielung einer möglichst geringen Masse ein möglichst geringes spezifisches Gewicht besitzt sowie zugleich auch für bildgebende oder bilderzeugende Medien, beispielsweise für Röntgenstrahlen, Magnetfelder oder elektromagnetische Wellen ein neutraler Werkstoff ist. Als Werkstoff für die als Hohlkörper ausgebildeten Arme wird vorzugsweise ein faserverstärkter, beispielsweise kohlefaserverstärkter Kunststoff verwendet.

Weiterbildungen, Vorteile und Anwendungsmöglichkeiten der Erfindung ergeben sich aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an einem Ausführungsbeispiel näher erläutert. Es zeigen:
- Fig. 1: in perspektivischer Darstellung die Verwendung eines erfindungsgemäßen Operations-Assistenz-Systems bei einem minimal invasiven Eingriff;
- Fig. 2: in perspektivischer Darstellung die Kinematik des erfindungsgemäßen Operations-Assistenz-Systems;
- Fig. 3: in Teildarstellung einen über ein Drehgelenk am äußeren Arm drehbar befestigten Instrumententräger und
- Fig. 4: in vereinfachter Schnittdarstellung das Drehgelenk zur drehbaren Verbindung des äußeren Arm mit dem Instrumententräger.

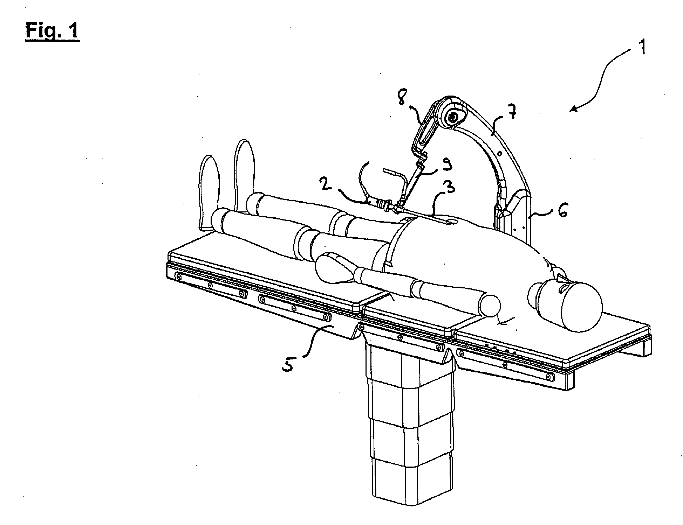

In Figur 1 ist allgemein mit 1 ein Operations-Assistenz-System für medizinische Interventionen bzw. Operationen zur Führung von chirurgischen Werkzeugen und Instrumenten, beispielsweise zum Führen eines mit einer Kamera 2 versehenen Endoskops 3, welches über eine kleinformatige Operationsöffnung ("Trokar") in einen Operationsraum innerhalb eines Patientenkörpers einführbar ist.

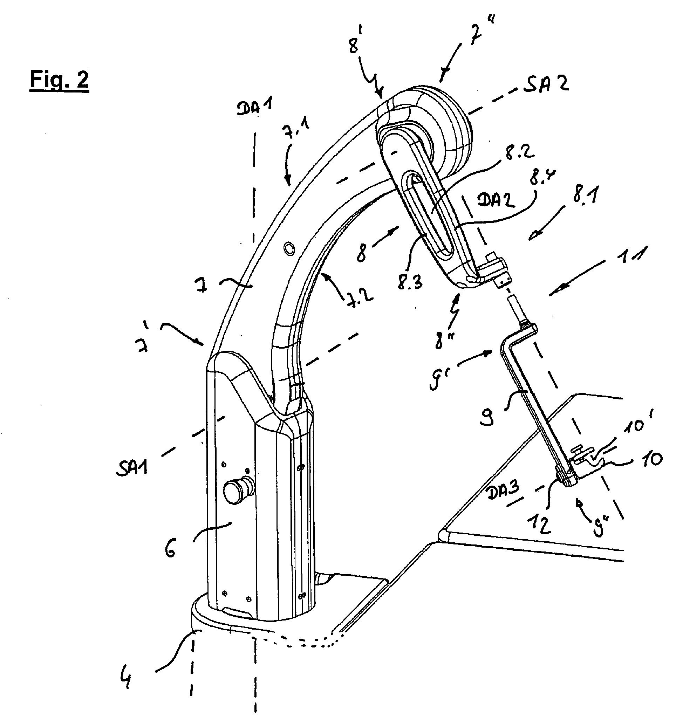

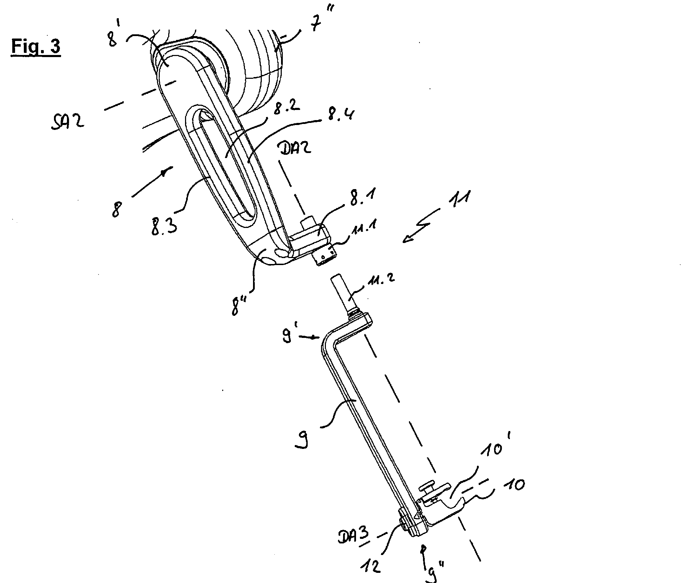

Das Operations-Assistenz-System 1 besteht im Wesentlichen aus einer Basis bzw. einem Gehäuse 4, in welchem die wesentlichen Funktions- und Steuerelemente des Operations-Assistenz-Systems 1 untergebracht sind und welches seitlich an einem Operationstisch 5 befestigbar ist. Zur Führung des chirurgischen Werkzeuges und Instrumentes, insbesondere des mit einer Kamera 2 versehenen Endoskops 3 weist das Operations-Assistenz-System 1 eine Kinematik bestehend aus wenigstens einer Tragsäule 6, zumindest einen inneren und äußeren Arm 7, 8 und einem Instrumententräger 9 auf, wobei der Instrumententräger 9 zur Aufnahme des zu führenden Endoskops 3 eine Instrumentenhalterung 10 aufweist. Der Aufbau der Kinematik ist in den Figuren 2 und 3 beispielhaft dargestellt.

Die Tragsäule 6 steht beispielsweise über die Oberseite des Gehäuses bzw. der Basis 4 vor und ist gesteuert drehbar um eine erste Drehachse DA1 an einer Basis 4 des Operations-Assistenz-Systems 1 vorgesehen, und zwar ist die Tragsäule 6 durch einen im Gehäuse 4 untergebrachten elektromotorischen Antrieb um die erste Drehachse DA1 ("Hochachse") zumindest gesteuert drehbar ausgebildet.

Im Bereich des oberen Ende der Tragsäule 6 ist an dieser das untere Ende 7' des inneren Armes 7 angelenkt und somit um eine erste Schwenkachse SA1 mit der Tragsäule 6 verbunden, vorzugsweise mittels eines motorisch steuerbaren Gelenks (nicht in den Figuren dargestellt). Der als Hohlkörper aus einem leichten, aber stabilen Werkstoff, z.B. aus faserverstärktem Kunststoff, beispielsweise aus glasfaserverstärktem oder kohlefaserverstärktem Kunststoff hergestellte innere Arm 7 ist in Armlängsrichtung leicht gekrümmt ausgebildet, und zwar an der Armoberseite 7.1 konvex und an der Armunterseite 7.2 konkav.

An dem der Tragsäule 6 entfernt liegenden oberen Ende 7" des inneren Armes 7 ist an diesem der äußere Arm 8 angelenkt, und zwar mit seinem oberen Ende 8'. Der innere Arm 7 ist über ein in den Figuren nicht dargestelltes ebenfalls motorisch steuerbaren Drehgelenk bestehend im Wesentlichen aus Lagerelementen und einem Gelenkbolzen mit dem oberen Ende 8' des äußeren Armes 8 verbunden, und zwar derart, dass der äußere Arm 8 schwenkbar um eine zweite Schwenkachse SA2 ausgebildet ist.

Der äußere Arm 8 ist ebenfalls als Hohlkörper aus einem leichten, aber stabilen Werkstoff, z.B. aus faserverstärktem Kunststoff, beispielsweise aus glasfaserverstärktem oder kohlefaserverstärktem Kunststoff hergestellt und ist im Querschnitt L-förmig ausgebildet, d.h. weist an seinem vom inneren Arm 7 beabstandeten unteren Ende 8" ein näherungsweise senkrecht zur Armlängsrichtung verlaufenden Trägerabschnitt 8.1 auf. Zur Gewichtsreduzierung weist der vorzugsweise flach ausgebildete äußere Arm 8 beispielsweise eine sich entlang der Armlängsrichtung erstreckende Öffnung 8.2 auf, so dass das obere und untere Ende 8', 8" des äußeren Armes 8 über zwei die längliche Öffnung 8.2 ausbildende Stegabschnitte 8.3, 8.4 miteinander verbunden sind. Die Längsachse der Trägerabschnittes 8.1 verläuft hierbei parallel bzw. näherungsweise parallel zur zweiten Schwenkachse SA2, wobei der Trägerabschnittes 8.1 in Richtung des angelenkten inneren Armes 7 seitlich wegsteht.

Am unteren Ende 8" des äußeren Armes 8, und zwar an dessen Trägerabschnitt 8.1 ist der Instrumententräger 9 mit seinem oberen Ende 9' drehbar um eine zweite Drehachse DA2 angelenkt, und zwar vorzugsweise mittels eines Drehgelenks 11. Somit ist der Instrumententräger 9 mit seinem oberen Ende 9' drehbeweglich mit dem unteren Ende 8" des äußeren Arms 8 verbunden, wobei erfindungsgemäß die zweite Drehachse DA2 näherungsweise senkrecht zur ersten und/oder zweiten Schwenkachse SA1, SA2 verläuft.

Ferner sind aufgrund der erfindungsgemäßen Anlenkung die Armlängsachse des äußeren Armes 8 und die Längsachse des Instrumententrägers 9 näherungsweise parallel zueinander vorgesehen. Am unteren Ende 9" der Instrumentenhalterung 9 ist die Instrumentenhalterung 10 angeordnet, an der das Endoskop 3 durch Verrasten lösbar befestigt ist und die ihrerseits mittels eines weiteren Drehgelenks 12 drehbar um eine dritte Drehachse DA3 an dem Instrumententräger 9 vorgesehen ist. Die dritte Drehachse DA3 verläuft beispielsweise senkrecht zur zweiten Drehachse DA2.

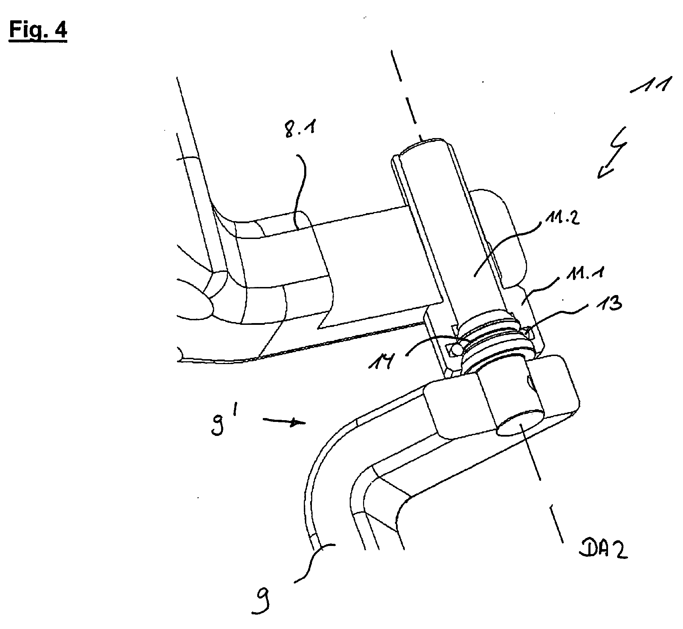

Das Drehgelenk 11 ist vorzugsweise zur lösbaren Befestigung des Instrumententrägers 9 am äußeren Arm 8 ausgebildet, und zwar vorzugsweise mit einem Schnellverschluss. Das Drehgelenk 11 weist hierzu beispielsweise zumindest eine Lagerhülse 11.1 und einen Gelenkbolzen 11.2 auf. Wie aus Figur 4 ersichtlich umfasst die die Lagerhülse 11.1 einen innen liegenden ein- oder mehrteilig ausgebildeten Sicherungsring 13, der in eine am Gelenkbolzen 11.2 vorgesehene ringförmige Nut 14 eingreift, so dass die in die Lagerhülse 11.1 im Gelenkbolzen 11.2 gehalten wird. Dadurch kann mittels einer entlang der zweiten Drehachse DA2 wirkenden Kraft der Gelenkbolzen 11.2 aus der Lagerhülse 11.1 schnell und einfach entnommen werden und somit der Instrumententräger 9 vom äußeren Arm 8 gelöst werden.

Bevorzugt ist die Instrumentenhalterung 10 derart am unteren Ende 9" der Instrumentenhalterung 9 angeordnet, dass die zweite Drehachse DA2 durch den Haltebereich 10' der Instrumentenhalterung 10 verläuft.

Die Drehgelenke 11 und 12 sind vorzugsweise als freie Gelenke ausgebildet, d.h. diese Gelenke ermöglichen ein freies Schwenken des Instrumententrägers 9 relativ zum äußeren Arm 8 bzw. der Instrumentenhalterung 10 relativ zum Instrumententräger 9.

Der Instrumententräger 9 ist vorzugsweise im Querschnitt L-förmig ausgebildet und weist somit einem ersten Trägerschenkel 9.1 auf, der in einen zweiten Trägerschenkel 9.2 übergeht, an dessen freien Ende 9" die Instrumentenhalterung 10 mittels des weiteren Drehgelenks 12 befestigt ist. Der erste und zweite Halteschenkel 9.1, 9.2 verlaufen näherungsweise senkrecht zueinander, wobei die mögliche Bewegungsbahn des zweiten Halteschenkels 9.2 näherungsweise eine Zylindermantelfläche beschreibt. Die Zylinderlängsachse fällt dabei mit der zweiten Drehachse DA2 zusammen.

Die Erfindung wurde voranstehend an einem Ausführungsbeispiel beschrieben. Es versteht sich, dass zahlreiche Änderungen sowie Abwandlungen möglich sind, ohne das dadurch der der Erfindung zugrunde liegende Erfindungsgedanke verlassen wird.

### Bezugszeichenliste

- 1: Operations-Assistenz-System
- 2: Kamera
- 3: Endoskop
- 4: Basis
- 5: Operationstisch
- 6: Tragsäule
- 7: innerer Arm
- 7': unteres Ende
- 7": oberes Ende
- 7.1: Armoberseite
- 7.2: Armunterseite
- 8: äußerer Arm
- 8': oberes Ende
- 8": unteres Ende
- 8.1: Trägerabschnitt
- 8.2: Öffnung
- 8.3: Stegabschnitt
- 8.4: Stegabschnitt
- 9: Instrumententräger
- 9': oberes Ende
- 9": unteres Ende
- 9.1: erster Trägerschenkel
- 9.2: zweiter Trägerschenkel
- 10: Instrumentenhalterung
- 11: Drehgelenk
- 11.1: Lagerhülse
- 11.2: Drehbolzen
- 12: weiteres Drehgelenk
- 13: Sicherungsring
- 14: ringförmige Nut

- DA1: erste Drehachse
- DA2: zweite Drehachse
- DA3: dritte Drehachse
- SA1: erste Schwenkachse
- SA2: zweite Schwenkachse

## Patentansprüche

1. Operations-Assistenz-System zur Führung von chirurgischen bzw. medizinischen Werkzeugen oder Instrumenten, beispielsweise zur Führung eines eine Kamera (2) aufweisenden Endoskops (3), mit einer eine Instrumentenhalterung (10) aufweisenden Kinematik, mit der das an der Instrumentenhalterung (10) befestigte chirurgische oder medizinische Werkzeug oder Instrument in mehreren Achsen motorisch und gesteuert bewegbar ist, wobei die Kinematik wenigstens eine Tragsäule (6), wenigstens einen inneren und äußeren Arm (7, 8) und wenigstens einen die Instrumentenhalterung (10) aufnehmenden Instrumententräger (9) umfasst, wobei die Tragsäule (6) gesteuert drehbar um eine erste Drehachse (DA1) an einer Basis (4) des Systems (1) vorgesehen ist, wobei das untere Ende (8") des äußeren Armes (8) mit dem der Instrumentenhalterung (10) gegenüberliegenden oberen Ende (9') des Instrumententrägers (9) drehbar um eine zweite Drehachse (DA2) verbunden ist und wobei das obere Ende (8') des äußeren Armes (8) über den inneren Arm (7) für eine gesteuerte Schwenkbewegung um wenigstens eine erste und zweite Schwenkachse (SA1, SA2) mit der Tragsäule (6) verbunden ist, wobei die zweite Drehachse (DA2) näherungsweise senkrecht zur ersten und/oder zweiten Schwenkachse (SA1, SA2) verläuft, **dadurch gekennzeichnet, dass** die Armlängsachse des äußeren Armes (8) parallel oder zumindest näherungsweise parallel zur Längsachse des Instrumententrägers (9) verläuft und zur drehbaren Verbindung des äußeren Armes (8) mit dem Instrumententräger (9) ein Drehgelenk (11) vorgesehen ist, das zur lösbaren Befestigung des Instrumententrägers (9) am äußeren Arm (8) ausgebildet ist.

2. Operations-Assistenz-System nach Anspruch 1, **dadurch gekennzeichnet, dass** der äußere Arm (8) im Querschnitt L-förmig ausgebildet ist.

3. Operations-Assistenz-System nach Anspruch 1 oder 2, **dadurch gekennzeichnet, dass** der vorzugsweise flach ausgebildete äußere Arm (8) zumindest zwei, das obere und untere Ende (8', 8") des äußeren Armes (8) miteinander verbindende Stegabschnitte (8.3, 8.4) aufweist, die eine sich entlang der Armlängsrichtung erstreckende Öffnung (8.2) einschließen.

4. Operations-Assistenz-System nach Anspruch 1, **dadurch gekennzeichnet, dass** das Drehgelenk (11) zumindest eine Lagerhülse (11.1) und einen Gelenkbolzen 11.2 aufweist.

5. Operations-Assistenz-System nach Anspruch 4, **dadurch gekennzeichnet, dass** zur lösbaren Befestigung die Lagerhülse (11.1) einen innen liegenden, ein- oder mehrteilig ausgebildeten Sicherungsring (13) aufweist, der in eine am Gelenkbolzen (11.2) vorgesehene ringförmige Nut (14) eingreift.

6. Operations-Assistenz-System nach Anspruch 4 oder 5, **dadurch gekennzeichnet, dass** der äußere Arm (8) einen Trägerabschnitt (8.1) zur Aufnahme der Lagerhülse (11.1) des Drehgelenks (11) aufweist.

7. Operations-Assistenz-System nach einem der vorhergehenden Ansprüche, **dadurch gekennzeichnet, dass** der Instrumententräger (9) L-förmig ausgebildet ist.

8. Operations-Assistenz-System nach Anspruch 7, **dadurch gekennzeichnet, dass** der Instrumententräger (9) einen ersten Trägerschenkel (9.1) aufweist, der in einen zweiten Trägerschenkel (9.2) übergeht, an dessen freien Ende (9") die Instrumentenhalterung (10) mittels eines weiteren Drehgelenks (12) befestigt ist.

9. Operations-Assistenz-System nach Anspruch 8, **dadurch gekennzeichnet, dass** der ersten Trägerschenkel (9.1) zur Aufnahme des Gelenkbolzens (11.2) des Drehgelenks (11) ausgebildet ist.

10. Operations-Assistenz-System nach einem der vorhergehenden Ansprüche, **dadurch gekennzeichnet, dass** zumindest der innere und/oder äußere Arm (7, 8) und/oder der Instrumententräger (24) als Hohlkörper aus einem Material hoher Festigkeit und geringer Masse ausgebildet sind.

11. Operations-Assistenz-System nach Anspruch 10, **dadurch gekennzeichnet, dass** das Material hoher Festigkeit und geringer Masse ein faserverstärkter Kunststoff, beispielsweise ein glasfaser- und/oder kohlefaserverstärkter Kunststoff ist.

## Claims

1. An operation assistance system for guiding surgical or medical tools or instruments, for example for guiding an endoscope (3) comprising a camera (2), having kinematics comprising an instrument holder (10) by means of which the surgical or medical tool or instrument mounted on the instrument holder (10) can be moved by motor and in controlled manner in a plurality of axes, wherein the kinematics comprise at least one support column (6), at least one inner and outer arm (7, 8) and at least one instrument carrier (9) accommodating the instrument holder (10), wherein the support column (6) is provided controlledly rotatable about a first axis of rotation (DA1) on a base (4) of the system (1), wherein the lower end (8") of the outer arm (8) is rotatably connected to the upper end (9') of the instrument carrier (9) opposite to the instrument holder (10) about a second axis of rotation (DA2), and wherein the upper end (8') of the outer arm (8) is connected with the support column (6) via the inner arm (7) for controlled pivotal movement about at least a first and a second pivotal axis (SA1, SA2), wherein the second axis of rotation (DA2) runs approximately perpendicular to the first and/or second pivotal axis (SA1, SA2), **characterized in that** the longitudinal axis of the outer arm (8) is parallel to or at least approximately parallel to the longitudinal axis of the instrument carrier (9) and a swivel joint (11) is provided for rotatably connecting the outer arm (8) with the instrument carrier (9), the swivel joint (11) being configured for removably mounting the instrument carrier (9) on the outer arm (8).

2. The operation assistance system according to claim 1, **characterized in that** the outer arm (8) is L-shaped in cross-section.

3. The operation assistance system according to claim 1 or 2, **characterized in that** the outer arm (8), which preferably has a flat configuration, comprises at least two web portions (8.3, 8.4) connecting the upper and lower end (8', 8") of the outer arm (8) to one another, the web portions enclosing an opening (8.2) which extends along the longitudinal direction of the arm.

4. The operation assistance system according to claim 1, **characterized in that** the swivel joint (11) comprises at least a bearing sleeve (11.1) and a joint bolt (11.2).

5. The operation assistance system according to claim 4, **characterized in that** for removable mounting, the bearing sleeve (11.1) comprises an inner single- or multiple-part retaining ring (13) which engages in a circular groove (14) provided in the joint bolt (11.2).

6. The operation assistance system according to claim 4 or 5, **characterized in that** the outer arm (8) comprises a carrier portion (8.1) to accommodate the bearing sleeve (11.1) of the swivel joint (11).

7. The operation assistance system according to one of the preceding claims, **characterized in that** the instrument carrier (9) has an L-shaped configuration.

8. The operation assistance system according to claim 7, **characterized in that** the instrument carrier (9) comprises a first carrier leg (9.1) merging into a second carrier leg (9.2) at the free end (9") of which the instrument holder (10) is mounted by means of a further swivel joint (12).

9. The operation assistance system according to claim 8, **characterized in that** the first carrier leg (9.1) is configured so as to accommodate the joint bolt (11.2) of the swivel joint (11).

10. The operation assistance system according to one of the preceding claims, **characterized in that** at least the inner and/or outer arm (7, 8) and/or the instrument carrier (24) is/are configured as a hollow body formed from a high strength low weight material.

11. The operation assistance system according to claim 10, **characterized in that** the high strength low weight material is a fibre-reinforced plastic, for example a glass fibre- and/or carbon fibre-reinforced plastic.

## Revendications

1. Système d'assistance à l'opération pour le guidage d'outils ou d'instruments chirurgicaux ou médicaux, par exemple le guidage d'un endoscope (3) présentant une caméra (2) comportant une cinématique présentant un support d'instrument (10) à l'aide duquel l'outil ou l'instrument chirurgical ou médical fixé au support d'instrument (10) est déplaçable de manière motorisée et contrôlée dans plusieurs axes, la cinématique comprenant au moins une colonne porteuse (6), au moins un bras intérieur et extérieur (7, 8) et au moins un porte-instrument (9) recevant le support d'instrument (10), la colonne porteuse (6) étant conçue de manière à pouvoir tourner autour d'un premier axe de rotation (DA1) au niveau d'une base (4) du système (1), l'extrémité inférieure (8") du bras extérieur (8) étant reliée à l'extrémité supérieure (9') opposée au support d'instrument (10) du porte-instrument (9) de manière à pouvoir tourner autour d'un second axe de rotation (DA2) et l'extrémité supérieure (8') du bras extérieur (8) étant reliée par l'intermédiaire du bras intérieur (7) pour un mouvement de pivotement contrôlé autour d'au moins un premier et second axes de pivotement (SA1, SA2) à la colonne porteuse (6), le second axe de rotation (DA2) s'étendant approximativement à la perpendiculaire du premier et/ou du second axe de rotation (SA1, SA2), **caractérisé en ce que** l'axe longitudinal de bras du bras extérieur (8) s'étend parallèlement ou du moins approximativement parallèlement à l'axe longitudinal du porte-instrument (9) et qu'il est prévu, pour la liaison avec possibilité de rotation du bras extérieur (8) au porte-instrument (9), un joint à rotule (11) qui est conçu pour la fixation dissociable du porte-instrument (9) au bras extérieur (8).

2. Système d'assistance à l'opération selon la revendication 1, **caractérisé en ce que** le bras extérieur (8) est réalisé avec une section transversale en forme de L.

3. Système d'assistance à l'opération selon la revendication 1 ou 2, **caractérisé en ce que** le bras extérieur (8) réalisé de préférence avec une conformation plate présente au moins deux sections de traverse (8.3, 8.4) reliant entre elles les extrémités supérieure et inférieure (8', 8") du bras extérieur (8) et qui circonscrivent un orifice (8.2) s'étendant suivant le sens longitudinal du bras.

4. Système d'assistance à l'opération selon la revendication 1, **caractérisé en ce que** le joint à rotule (11) présente au moins un coussinet de palier (11.1) et un goujon d'articulation (11.2).

5. Système d'assistance à l'opération selon la revendication 4, **caractérisé en ce que**, en vue d'une fixation dissociable, le coussinet de palier (11.1) présente une bague de blocage (13) située à l'intérieur et réalisée en une ou plusieurs pièces qui s'engrène dans une rainure (14) de forme annulaire prévue au niveau du goujon d'articulation (11.2).

6. Système d'assistance à l'opération selon la revendication 4 ou 5, **caractérisé en ce que** le bras extérieur (8) présente une section porteuse (8.1) destinée à recevoir le coussinet de palier (11.1) du joint à rotule (11).

7. Système d'assistance à l'opération selon une des revendications précédentes, **caractérisé en ce que** le porte-instrument (9) est réalisé en forme de L.

8. Système d'assistance à l'opération selon la revendication 7, **caractérisé en ce que** le porte-instrument (9) présente une première branche porteuse (9.1) qui transite dans une seconde branche porteuse (9.2) à l'extrémité libre (9") de laquelle le support d'instrument (10) est fixé au moyen d'un autre joint à rotule (12).

9. Système d'assistance à l'opération selon la revendication 8, **caractérisé en ce que** la première branche porteuse (9.1) est conçue pour recevoir le goujon d'articulation (11.2) du joint à rotule (11).

10. Système d'assistance à l'opération selon une des revendications précédentes, **caractérisé en ce qu'**au moins le bras intérieur et/ou extérieur (7, 8) et/ou le porte-instrument (24) sont réalisés sous forme d'un corps creux en matériau à haute résistance et à faible masse.

11. Système d'assistance à l'opération selon la revendication 10, **caractérisé en ce que** le matériau à haute résistance et à faible masse est un plastique renforcé aux fibres, par exemple un plastique renforcé aux fibres de verre et/ou de carbone.
